(19) Europäisches Patentamt
European Patent Office
Office européen des brevets

(11) Veröffentlichungsnummer: **0 339 394 B1**

# (12) EUROPÄISCHE PATENTSCHRIFT

(45) Veröffentlichungstag der Patentschrift: **27.12.91 Patentblatt 91/52**

(51) Int. Cl.[5]: **C07C 319/24**, C07C 321/14

(21) Anmeldenummer: **89106693.8**

(22) Anmeldetag: **14.04.89**

## (54) Verfahren zur Herstellung von Disulfiden.

(30) Priorität: **27.04.88 DE 3814163**

(43) Veröffentlichungstag der Anmeldung: **02.11.89 Patentblatt 89/44**

(45) Bekanntmachung des Hinweises auf die Patenterteilung: **27.12.91 Patentblatt 91/52**

(84) Benannte Vertragsstaaten: **AT BE CH DE ES FR GB GR IT LI LU NL SE**

(56) Entgegenhaltungen: **HOUBEN-WEYL, Methoden der Organischen Chemie, Band E11, Seiten 135-142, herausgegeben von K.H. BÜCHEL et al., Georg Thieme Verlag, Stuttgart, DE; R. BECKERT et al.: "Organische Schwefel-Verbindungen"**

(73) Patentinhaber: **RHEIN-CHEMIE RHEINAU GMBH**
**Postfach 81 04 09 Mülheimer Strasse 24-28**
**W-6800 Mannheim 81 (DE)**

(72) Erfinder: **Sauerbier, Michael, Dr.**
**Lochwiesenstrasse 4**
**W-6701 Altrip (DE)**
Erfinder: **Nützel, Karl, Dr.**
**Kornstrasse 23**
**W-6823 Neulussheim (DE)**
Erfinder: **Schilling, Kurt**
**Karl-Strasse 45**
**W-6830 Schwetzingen (DE)**

(74) Vertreter: **Gremm, Joachim, Dr. et al**
**Bayer AG Konzernverwaltung RP Patente Konzern**
**W-5090 Leverkusen 1, Bayerwerk (DE)**

EP 0 339 394 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Diorganodisulfide und ihre Herstellung durch Oxidation entsprechender Mercaptane sind bekannt, vgl. Houben-Weyl, Methoden der organischen Chemie, Bd. C11/1, S. 133, Georg Thieme Verlag, 1985. Als Oxidationsmittel hat man beispielsweise Sauerstoff, Wasserstoffperoxid, Chlor, Brom, Iod, Hypochlorit, Hypobromit, Hypoiodit, Eisen (III) chlorid, Kaliumhexacyanoferrat-III und Stickoxid verwendet. Alle diese Oxidationsmittel sind jedoch unbefriedigend. Sie liefern entweder mäßige Ausbeuten oder die Reaktion ist schlecht beherrschbar (z.B. bei der Verwendung von Wasserstoffperoxid) oder es treten Nebenprodukte in erheblichen Mengen auf (z.B. bei Verwendung elementarer Halogene und Wasserstoffperoxid).

Gegenstand der Erfindung ist ein Verfahren zur Herstellung von Diorganodisulfiden durch Oxidation von Mercaptanen, das dadurch gekennzeichnet ist, daß man das Mercaptan in Gegenwart einer Base und gegebenenfalls eines Phasentransferkatalysators bei Temperaturen von 0 bis 100°C mit einer Halogenverbindung umsetzt, die in der Lage ist, mindestens ein Halogenatom abzugeben und dafür ein Wasserstoffatom aufzunehmen.

Bevorzugt wird das Verfahren in wäßrigem Medium durchgeführt. Die Reaktion verläuft dann in heterogener Phase. Es ist auch möglich, in homogener Phase, z.B. in einem organischen Lösungsmittel oder ohne Lösungsmittel zu arbeiten. Geeignet sind als Lösungsmittel insbesondere Alkohole oder die zur Oxidation verwendeten Halogenverbindungen selbst. Bei der Umsetzung in homogener Phase werden als Basen tertiäre Amine bevorzugt. Die Base verwendet man im allgemeinen in Mengen von mindestens 0.5 Mol pro Mol Mercaptan. Ein Überschuß an Base beeinflußt die Reaktion nicht. Der Phasentransferkatalysator wird in katalytisch wirksamen Mengen eingesetzt, im allgemeinen etwa 0,5 bis 10 Millimol pro Mol Mercaptan. Die Halogenverbindung kann im Überschuß verwendet werden, meist genügen aber etwa stöchiometrische Mengen, d.h. ungefähr 0,5 Mol, bevorzugt 0,3 bis 0,7 Mol, pro Mol Mercaptan.

Durch das erfindungsgemäße Verfahren werden Diorganodisulfide in hoher Ausbeute und hoher Reinheit unter sehr milden Reaktionsbedingungen erhalten.

Die erfindungsgemäße Umsetzung läßt sich durch die folgende Reaktionsgleichung beispielhaft veranschaulichen :

$$2\ R\text{-}SH + CCl_4 + NaOH \xrightarrow[\text{katalysator}]{\text{Phasentransfer-}} R\text{-}S\text{-}S\text{-}R + HCCl_3 + NaCl + H_2O$$

Das erfindungsgemäße Verfahren kann im allgemeinen so durchgeführt werden, daß man das Mercaptan, die Base und den Phasentransferkatalysator in einem wäßrigen Medium oder einem organischen Medium oder auch ohne Lösungsmittel vorlegt, und daß man die Halogenverbindung langsam zugibt. Man kann die Umsetzung bei Zimmertemperatur beginnen, sie springt sofort an und verläuft exotherm, so daß die Zugabegeschwindigkeit benutzt werden kann, um die Reaktionstemperatur zu steuern. Selbstverständlich kann das Reaktionsgemisch im Bedarfsfall gekühlt oder auch erwärmt werden. Soweit es sich um ein heterogenes System handelt, ist es erforderlich, während der Reaktion das Reaktionsgemisch in Bewegung zu halten, in den anderen Ausführungsformen ist dies empfehlenswert, z.B. durch Rühren. Am Ende der Reaktion kann man, wenn man in wäßrigem Medium arbeitet, die wäßrige Phase von der organischen Phase abtrennen und die im wesentlichen aus Diorganodisulfid und Reduktionsprodukt der Halogenverbindung bestehende organische Phase in an sich bekannter Weise, z.B. Destillation, reinigen. Arbeitet man in homogener Phase, dann kann man von dem gebildeten Amin-Hydrochlorid abtrennen, und die organische Phase z.B. destillativ aufarbeiten.

Im allgemeinen führt man die Reaktion bei Temperaturen von 0 bis 100°C, bevorzugt 20 bis 65°C, und insbesondere 20 bis 35°C durch. Die erfindungsgemäß hergestellten Diorganodisulfide sind meist bei Zimmertemperatur flüssig, oder sie sind niedrigschmelzende Feststoffe.

Für das Verfahren geeignete Mercaptane sind in erster Linie Alkylmercaptane (geradkettig oder verzweigt), bevorzugt mit 1 bis 18, besonders bevorzugt 1 bis 12 C-Atomen. Ebenfalls geeignet sind Aralkylmercaptane wie Benzylmercaptan oder substituierte Mercaptane wie Mercaptoalkanole (Mercaptoethanol) oder Mercaptocarbonsäureester wie Mercaptopropionsäureester, Mercaptoessigsäureester. Auch Mercaptane mit mehreren SH-Gruppen, z.B. $\alpha,\omega$-Dimercaptoalkane mit bevorzugt 2 bis 6 C-Atomen wie 1,2-Dimercaptoethan, 1,3-Dimercaptopropan sowie β-Mercaptopropionsäure-tris-trimethylolpropanester und β-Mercaptopropionsäure-tetra-pentaerythritester sind geeignet. Aus solchen Ausgangsprodukten entstehen Disulfide mit mehreren -S-S-Gruppen, bis hin zu Polymeren. Durch Mitverwendung von monofunktionellen Mercaptanen kann man die Kettenlänge solcher Produkte regeln. Selbstverständlich können auch Mischungen von Mercaptanen umgesetzt werden. Hieraus ergibt sich, daß der Rest R in der oben angegebenen Reaktionsgleichung bevorzugt ein Alkylrest, insbesondere mit 1 bis 18 oder 1 bis 12 C-Atomen ist, sowie ein entsprechender Hydroxyalkylrest oder auch ein Carbonsäurerest. Die beiden Reste R können auch verschieden sein. Die Möglichkeiten der Unterschiede ergeben sich aus den oben angeführten unterschiedlichen Mercaptoverbindungen.

Die die Oxidation der Mercaptane bewirkenden Halogenverbindungen müssen in der Lage sein, ein Halogenatom abzugeben und ein Wasserstoffatom aufzunehmen. Geeignet sind beispielsweise Tetrachlormethan, Tetrabrommethan, Trichlorbrommethan, Bromoform, Iodoform, 1,1-Dichlor-2-brommethan, symmetrisches Tetrachlordibrommethan, Pentachlorethan, Hexachlorethan, Octachlorpropan, Decachlorbutan. Verallgemeinert handelt es sich also um halogenierte, insbesondere chlorierte, bromierte oder iodierte, bevorzugt gesättigte Kohlenwasserstoffe mit 1 bis 6 C-Atomen, die im allgemeinen perhalogeniert sind oder jedenfalls nur noch ein oder zwei Wasserstoffatome enthalten.

Als Basen können anorganische Basen wie Alkalihydroxide, Alkalicarbonate, Erdalkalihydroxide verwendet werden oder organische Basen wie tertiäre Amine. Sie können fest oder als wäßrige Lösungen verwendet werden. Beispiele sind Natriumhydroxid, Kaliumhydroxid, Natriumcarbonat, Kaliumcarbonat, Bariumhydroxid, Calciumhydroxid, Triethylamin, N,N-Dimethylcyclohexylamin, N,N-Dimethylbenzylamin. Natriumhydroxid ist bevorzugt.

Als Phasentransferkatalysatoren werden bevorzugt quartäre Ammoniumsalze und quartäre Phosphoniumsalze verwendet. Beispiele sind Tetrabutylammoniumbromid, Benzyltrimethylammoniumchlorid, Tricaprylmethylammoniumchlorid, Benzyltriphenylphosphoniumbromid, Tributylhexadecylphosphoniumbromid.

Durch die folgenden Beispiele wird das erfindungsgemäße Verfahren näher erläutert.

Beispiel 1

In einen Dreihalskolben ausgerüstet mit Rührer, Rückflußkühler und Tropftrichter werden 160 g (1 Mol) tert.-Nonylmercaptan 22 g (0,55 Mol) Natriumhydroxid, 75 ml Wasser und 0,8 g (2 mMol) Tricaprylmethylammoniumchlorid eingebracht.

Unter Rühren und Wasserkühlung läßt man innerhalb einer Stunde 77 g (0,5 Mol) Tetrachlormethan zutropfen, wobei die Temperatur von 20 auf 30°C ansteigt. Man läßt noch 2 Stunden nachrühren. Anschließend wird die wäßrige Phase von der organischen Phase in einem Scheidetrichter getrennt. Die organische Phase wird im Wasserstrahlvakuum bei 90°C von dem gebildeten Chloroform und der Restfeuchtigkeit befreit und abschließend filtriert.

Ausbeute an Di-tert.-Nonyldisulfid : 155,5 g (98,4%).

Beispiel 2

Wie in Beispiel 1 beschrieben, werden 202 g (1 Mol) n-Dodecylmercaptan, 22 g (0,55 Mol) Natriumhydroxid, 100 ml Wasser und 0,6 g (1,5 mMol) Tricaprylmethylammoniumchlorid mit 77 g (0,5 Mol) Tetrachlormethan umgesetzt.

Ausbeute an Di-n-Dodecyldisulfid : 198 g (99%).

Beispiel 3

Wie in Beispiel 1 beschrieben, werden 720 g (8 Mol) tert.-Butylmercaptan, 501,3 g (5,33 Mol) 1,2-Ethandithiol, 392 g (9,8 Mol) Natriumhydroxid, 1.330 ml Wasser und 3,1 g (7,75 mMol) Tricaprylmethylammoniumchlorid mit 1.437,3 g (9,33 Mol) Tetrachlormethan umgesetzt.

Ausbeute an "gemischtem Disulfid" der Formel I : 1.178 g (98,6%)

$$\text{t-Butyl-S-S}\left[\text{CH}_2\text{-CH}_2\text{-S-S}\right]_{1,3}\text{t-Butyl}$$

Formel I

## Patentansprüche

1. Verfahren zur Herstellung von Diorganodisulfiden durch Oxidation von Mercaptanen, dadurch gekennzeichnet, daß man das Mercaptan in Gegenwart einer Base und gegebenenfalls eines Phasentransferkatalysators bei Temperaturen von 0 bis 100°C mit einer Halogenverbindung umsetzt, die in der Lage ist, mindestens ein Halogenatom abzugeben und dafür ein Wasserstoffatom aufzunehmen.

2. Verfahren nach Anspruch 1, dadurch gekennzeichnet, daß man als Mercaptan ein $C_1$-$C_{18}$-Alkylmercaptan verwendet.

3. Verfahren nach Anspruch 1, dadurch gekennzeichnet, daß man als Mercaptan ein α,ω-Dimercaptoalkan mit 1 bis 6 C-Atomen gegebenenfalls zusammen mit einem $C_1$-$C_{18}$-Alkylmercaptan verwendet.

4. Verfahren nach Anspruch 1, dadurch gekennzeichnet, daß man als Halogenverbindung Tetrachlorkohlenstoff verwendet.

5. Verfahren nach Anspruch 1, dadurch gekennzeichnet, daß man als Base Natriumhydroxid verwendet.

6. Verfahren nach Anspruch 1, dadurch gekennzeichnet, daß man in wäßrigem Medium arbeitet.

## Claims

1. A process for the production of diorganodisulfides by oxidation of mercaptans, characterized in that the mercaptan is reacted with a halogen compound which is capable of releasing at least one halogen atom and taking up a hydrogen atom in its place, the reaction being carried out at a temperature of 0 to 100°C in the presence of a base and, optionally, a phase transfer catalyst.

2. A process as claimed in claim 1, characterized in that a $C_1$-$C_{18}$ alkyl mercaptan is used as the mercaptan.

3. A process as claimed in claim 1, characterized in that an α,ω-dimercaptoalkane containing 1 to 6 C atoms, optionally together with a $C_1$-$C_{18}$ alkyl mercaptan, is used as the mercaptan.

4. A process as claimed in claim 1, characterized in that carbon tetrachloride is used as the halogen compound.

5. A process as claimed in claim 1, characterized in that sodium hydroxide is used as the base.

6. A process as claimed in claim 1, characterized in that the reaction is carried out in aqueous medium.

## Revendications

1. Procédé de préparation de diorganodisulfures par oxydation de mercaptans, caractérisé en ce que l'on fait réagir les mercaptans en présence d'une base et le cas échéant d'un catalyseur à transfert de phase, à des températures de 0 à 100°C, avec un dérivé halogéné capable de fournir au moins un atome d'halogène en absorbant un atome d'hydrogène.

2. Procédé selon la revendication 1, caractérisé en ce que le mercaptan mis en oeuvre est un alkylmercaptan en $C_1$-$C_{18}$.

3. Procédé selon la revendication 1, caractérisé en ce que le mercaptan mis en oeuvre est un α,ω-dimercapto-alcane en $C_1$-$C_6$, éventuellement avec un alkylmercaptan en $C_1$-$C_{18}$.

4. Procédé selon la revendication 1, caractérisé en ce que le dérivé halogéné utilisé est le tétrachlorure de carbone.

5. Procédé selon la revendication 1, caractérisé en ce que la base utilisée est l'hydroxyde de sodium.

6. Procédé selon la revendication 1, caractérisé en ce que l'on opère en milieu aqueux.